# EUROPÄISCHE PATENTSCHRIFT

(11) **EP 2 974 747 B1**
(45) Veröffentlichungstag und Bekanntmachung des Hinweises auf die Patenterteilung: **09.08.2017**
(21) Anmeldenummer: 15175564.2
(22) Anmeldetag: 14.11.2013
(51) Int. Cl.: A61L 2/10, A61L 9/20, C02F 1/32, F24D 5/08, B01D 53/00, G01J 3/02

(54) **KOMPAKTES SYSTEM MIT HOHER HOMOGENITÄT DES STRAHLUNGSFELDS**
COMPACT SYSTEM WITH HIGH HOMOGENEITY OF THE RADIATION FIELD
SYSTÈME COMPACT À HAUTE HOMOGÉNÉITÉ DU CHAMP DE RAYONNEMENT

(30) Priorität: 15.11.2012 DE 102012022326
(43) Veröffentlichungstag der Anmeldung: 20.01.2016
(62) Teilanmeldung aus: 13795689.2
(73) Patentinhaber: SCHOTT AG, 55122 Mainz (DE)
(72) Erfinder: Blechschmidt, Jörg, 55270 Zornheim (DE); Kluge, Michael, 63071 Offenbach (DE); Plapper, Volker, 55232 Alzey (DE)
(74) Vertreter: Sawodny, Michael-Wolfgang

(56) Entgegenhaltungen:
- EP-A2- 0 351 518
- WO-A1-2004/011915
- WO-A1-2011/075694
- WO-A2-02/14925
- WO-A2-2007/027419
- DE-A1-102004 021 585
- GB-A- 2 334 873
- US-A- 5 247 178
- US-A1- 2005 115 498

## Beschreibung

Die Erfindung betrifft ein kompaktes System mit hoher Homogenität des Strahlungsfelds, verglichen mit Anordnungen, die aus dem Stand der Technik bekannt sind.

Es ist bereits bekannt Strahlung, insbesondere UV- oder IR-Strahlung, zur Behandlung von Wasser, Gasen, insbesondere Luft, oder Oberflächen einzusetzen. Insbesondere ist die Desinfektion mit UV-Strahlung bekannt. Relativ verbreitet ist bislang die Trinkwasseraufbereitung mit UV-Strahlung, wobei die Keimzahl im Wasser zuverlässig und in Abhängigkeit zur Dosis stark reduziert werden kann. Durch die UV-Strahlung werden beispielsweise Mikroorganismen, wie Krankheitserreger, insbesondere Bakterien oder Viren, inaktiviert.

Der Wirkungsgrad eines Behandlungssystems wird in hohem Maße durch die Homogenität des erzeugten Strahlungsfelds, in dem sich das zu behandelnde Medium, wie beispielsweise Wasser befindet, bestimmt. Insbesondere bei Systemen mit wenigen Lichtquellen ist die Erzielung einer ausreichenden Homogenität schwierig und meist mit hohen Leistungsverlusten behaftet. Für die Behandlungsleistung ist es daher bevorzugt eine möglichst homogene Verteilung der Strahlungsintensität bereitzustellen. Dabei ist eine lokale Erhöhung der Intensität nicht schädlich. Eine lokal stark reduzierte Intensität kann aber dazu führen, dass eine unzureichende Behandlung vorliegt. Im Falle einer Desinfektion durch UV-Strahlung werden beispielsweise Keime, die beim Durchfluss durch einen UV-Reaktor durch diese Bereiche fließen, aufgrund der geringen Strahlungsintensität nicht genügend bestrahlt, und daher nicht ausreichend inaktiviert.

Weiterhin ist für Anwendungen in Bereichen mit starken räumlichen Beschränkungen eine kompakte Ausführung des Behandlungssystems wichtig, ohne jedoch Kompromisse bei der Systemeffizienz einzugehen. Zudem muss aus Platzgründen und meist auch Kostenaspekten die Anzahl der Strahlquellen so weit wie möglich reduziert werden.

Aus dem Stand der Technik sind beispielsweise zwei konzeptionelle Ansätze zur UV-Desinfektion bekannt geworden:

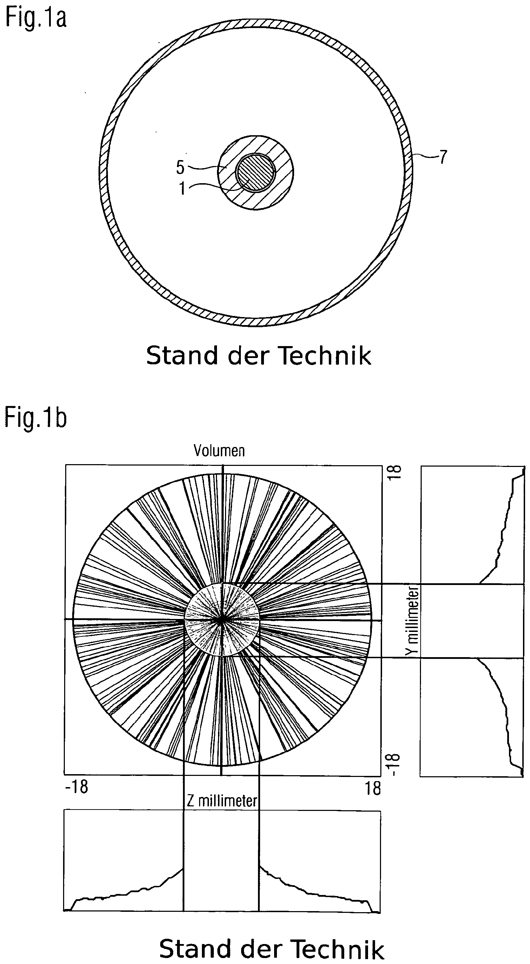

Beim ersten Konzept wird eine hohe Kompaktheit der Anordnung erreicht, wobei jedoch gleichzeitig die Strahlungshomogenität leidet. Ein typisches Beispiel einer derartigen Anordnung ist die Koaxialgeometrie. Beispielhaft ist in Fig. 1 a eine Ausführungsform eines derartigen Systems dargestellt, wie dieses aus dem Stand der Technik bekannt ist. Fig. 1 a zeigt eine Draufsicht auf eine stabförmige UV-Lichtquelle 1, die sich senkrecht zur Zeichenebene erstreckt, innerhalb eines Rohrs 7 angeordnet ist und von einem Medium, wie Wasser, umflossen wird. Dabei wird die UV-Lichtquelle 1 durch ein UV-transparentes Hüllrohr 5 vor dem Wasser geschützt. Da die Strahlungsintensität der UV-Lichtquelle quadratisch mit dem Abstand abnimmt und zusätzlich durch Absorption im Medium geschwächt wird, ergibt sich in der Fig. 1a ein inhomogenes Strahlungsfeld.

Um das inhomogene Strahlungsfeld von Fig. 1a zu verdeutlichen, wird die Art des Strahlungsfelds, die sich für eine Anordnung gemäß Fig. 1 a ergibt, in Fig. 1 b anhand einer Simulation mit der sogenannten Ray Tracing-Methode im Einzelnen gezeigt. Bei der Ray Tracing-Methode werden von der Strahlungsquelle ausgehende Strahlengänge berechnet, wobei die optischen Parameter der durchdrungenen Materialien, insbesondere Absorptions- und Reflexionskoeffizienten, berücksichtigt werden. Durch die Berechnung einer hohen Anzahl statistisch erzeugter Ausgangsstrahlen wird das resultierende Strahlungsfeld abgebildet. Dieses Verfahren ist dem Fachmann aus dem Stand der Technik bekannt und muss daher nicht näher erläutert werden.

In Fig. 1b ist gezeigt wie sich das Strahlungsfeld in der Anordnung von Fig. 1 a darstellt, wobei die einzelne UV-Lichtquelle 1 mittig innerhalb des Rohrs 7 angeordnet vorliegt. Die beiden Diagramme am unteren und rechten Rand in Fig. 1b zeigen jeweils den Verlauf der Strahlungsdichte im Schnitt. Das untere Diagramm zeigt die Strahlungsintensität im horizontalen Schnitt durch die Zeichnungsmitte (z Millimeter) und das rechte Diagramm zeigt die Strahlungsintensität im vertikalen Schnitt durch die Zeichnungsmitte (y Millimeter). Nicht Medium-führende Bereiche wurden bei der Darstellung ausgeblendet. Die Diagramme veranschaulichen daher die Strahlungsintensität entlang der gewählten Schnittebenen. Ein perfekt homogenes Strahlungsfeld würde in einer flachen, horizontalen Linie resultieren (sog. "Hutprofil"). Ein stark inhomogenes Strahlungsfeld resultiert in einer starken Abweichung der Werte entlang des gewählten Schnitts. Aus Fig. 1b geht daher hervor, dass die Strahlungsintensität in der Nähe der UV-Lichtquelle maximal ist und zum äußeren Rand des Rohrs hin stark abfällt. Für die Anordnung von Fig. 1 a wurde gemäß Fig. 1b eine Standardabweichung vom Mittelwert der Strahlungsdichte von 43% berechnet. Ein derartig großer Wert belegt eine schlechte Strahlungshomogenität des Systems. Demzufolge weist die Anordnung von Fig. 1 a zwar eine hohe Kompaktheit auf, jedoch ein sehr inhomogenes Strahlungsfeld. Ein stark inhomogenes Strahlungsfeld bedeutet in diesem Fall jedoch, dass Bereiche vorliegen, in denen vorhandene Keime, die beim Durchfluss durch das Rohr 7 durch diese Bereiche fließen, aufgrund der geringen Strahlungsintensität nicht genügend bestrahlt werden, um ausreichend inaktiviert zu werden. Die Desinfektionsleistung ist daher unzureichend.

Weitere beispielhafte Vorrichtungen zur Desinfektion aus dem Stand der Technik, die ebenfalls relativ kompakt aufgebaut sind, aber über unzureichende Strahlungshomogenität verfügen, sind die Folgenden:

Die US 2007/0272877 A1 bezieht sich auf eine Bestrahlungsvorrichtung, insbesondere UV-Desinfektionsvorrichtung, umfassend mindestens einen Reaktor zur Behandlung von Fluiden mit Lichtstrahlung, wobei der Reaktor ein Rohr bzw. einen Kanal oder einen Behälter aus transparentem Material umfasst und von Luft umgeben ist, mit einen Fluideinlass, einen Fluidauslass und mindestens einer Öffnung oder einem Fenster, die/das für die Transmission von Licht in das Rohr bzw. den Kanal angepasst ist. Außerhalb des Rohrs bzw. Kanals befindet sich eine Lichtquelle, die einen Lichtgenerator und einen Reflektor aufweist, um Licht, das vom Lichtgenerator erzeugt wird, in Richtung des Fensters in einem vordefinierten Winkelbereich reflektiert. Insbesondere ist hier ein zylinderförmiger Reaktor vorgesehen, der zumindest teilweise so ausgebildet sein kann, dass auf die Wände auftreffendes Licht, insbesondere UV-Licht, in das Medium zurückreflektiert wird.

Weiterhin bezieht sich die US 6 337 483 B1 auf eine keimtötende UV-Kammer zur Verwendung bei Luft, wobei die UV-Kammer selbst als Reflektor ausgebildet sein kann und bevorzugt die Form eines beidseitig abgeschnittenen Ellipsoids aufweist.

Die Offenbarung der US 6 555 011 B1 bezieht sich auf ein Verfahren zur Desinfektion und Reinigung von Flüssigkeiten und Gasen, wobei ein spezielles Reaktordesign eingesetzt wird, bei dem die reflektierenden Seitenwände zur Konzentrierung der UV-Strahlung während der Desinfektion von Flüssigkeiten und Gasen beitragen.

Ferner bezieht sich die US 2010/0264329 A1 auf eine Desinfektionsvorrichtung für Flüssigkeiten mit Hilfe von Licht, wobei die Vorrichtung umfasst: Ein im Wesentlichen Licht-transparentes Rohr, um die hindurchfließende Flüssigkeit zu desinfizieren, eine im Wesentlichen Licht-transparente Umhüllung mit Außendimensionen, die kleiner sind als die Innendimensionen des Rohrs, wobei die Umhüllung im Rohr im Wesentlichen senkrecht zur Symmetrieachse des Rohrs angeordnet ist, sowie eine Lichtquelle, die innerhalb der Umhüllung angeordnet ist. Bevorzugt dient ein Rohr aus Quarzglas als Reaktor und befindet sich innerhalb von reflektierenden Wänden eines Reflektors.

Erwähnt sei auch die US 5 216 251 A, die eine Desinfektions- und Trocknungsvorrichtung für Hände und Unterarme beschreibt, wobei in einer Arbeitskammer UV-Licht eingesetzt wird, um vorerwärmte Luft aus einer zweiten Kammer, die mit der Arbeitskammer verbunden ist, zu desinfizieren, und dann hiermit die Hände bzw. Arme in einer geschlossenen Kammer zu desinfizieren und zu trocknen. Diese Vorrichtung hat eigentlich eine andere Ausrichtung als die vorliegende Erfindung, da das desinfizierte Medium in Form von Luft zur Desinfektion und Trocknung der Hände eingesetzt und daher in einem mehr oder weniger abgeschlossenen Raum desinfiziert wird.

Beim zweiten konzeptionellen Ansatz gemäß dem Stand der Technik werden UV-Desinfektionssysteme zur Verfügung gestellt, die zwar ein relativ homogenes Strahlungsfeld erzeugen, aber hierfür einen außerordentlich großen Bauraum benötigen, d.h. nicht ausreichend kompakt ausgelegt sind:

So beschreibt beispielsweise die GB 2 334 873 A eine Sterilisierungsvorrichtung, umfassend eine Vielzahl von elliptischen Reflektoren. In Figur 1 der GB 2 334 873 A ist ein elliptischer Doppelreflektor 1 um eine Probenröhre 2 angeordnet, wobei die Probenröhre am gemeinsamen Brennpunkt des Reflektors angeordnet ist. Zwei Quecksilberlampen 3 sind an den anderen beiden Brennpunkten des elliptischen Doppelreflektors 1 positioniert.

Weiterhin offenbart die US 5 247 178 A eine Vorrichtung zur Behandlung eines Fluids durch Bestrahlung eines dünnen Films des Fluids mit konzentriertem Licht hoher Intensität. Zur Bestrahlung wird eine ringförmige Leitung 102 vorgesehen, damit ein dünner Film des Fluids vorliegt, der bestrahlt wird. Im Inneren wird die ringförmige Leitung 102 von einem Schaft 103 begrenzt, dessen Oberfläche reflektiv ausgestaltet ist. Nach Außen wird die ringförmige Leitung 102 von einer transparenten Leitung 104 umgeben. Es wird ein elliptischer reflektiver Zylinder 101 vorgesehen, wobei eine Strahlungsquelle im oder in der Nähe des ersten Brennpunkts des elliptischen Zylinders angeordnet wird und das zu bestrahlende Medium im oder in der Nähe des zweiten Brennpunkts angeordnet wird, wie im Einzelnen aus Figur 1 der US 5 247 178 A hervorgeht.

In der Lehre der GB 2 334 873 A als auch der US 5 247 178 A sind demnach die UV-Lichtquellen außerhalb des UV-Reaktors angeordnet. Über eine Anordnung von außen liegenden Reflektoren wird die UV-Strahlung möglichst gleichmäßig durch die UV-transparente Reaktorwand in das Medium eingekoppelt. Heute bekannte Systeme nutzen dazu Reflektoren, deren Spiegelflächen in der Regel von der UV-transparenten Reaktorwand getrennt sind. Das UV-Licht wird außerhalb des Medium-führenden UV-Reaktors so verteilt, dass sich als Konsequenz ein möglichst homogenes Strahlungsfeld innerhalb des UV-Reaktors ergeben soll.

Aus dem Stand der Technik gemäß der DE 38 24 647 A1 ist auch eine Vorrichtung zur Bestrahlung von Medien mittels UV-Licht bekannt, bestehend aus einem vom Medium durchströmten Rohrkörper aus UV-durchlässigem Werkstoff und mindestens zwei außen achsparallel angeordneten UV-Lichtquellen mit Reflektoren, wobei die Lichtquellen UV-Flachstrahler darstellen, die einen länglich, flachovalen Querschnitt mit Breit- und Schmalseite aufweisen, wobei die Hauptachsen der UV-Lichtquellen jeweils auf den Mittelpunkt des Rohrkörperquerschnitts gerichtet sind. Die UV-Lichtquellen sind kranzförmig und achsparallel um den Medium-durchströmten Rohrkörper angeordnet. Gemäß einer Ausführungsform liegen die Flachstrahler mit der dem Rohrkörper zugewandten Schmalseite am Rohrkörper an. Im Gegensatz zur erfindungsgemäßen Lehre ist der UV-Reaktor nicht als Reflektor ausgebildet. Die Reflektoren sind ausschließlich den UV-Lichtquellen zugeordnet und bilden keinen Teil des UV-Reaktors selbst aus, in dem das zu desinfizierende Medium strömt. Erfindungsgemäß wird demgegenüber der das zu verwendende/zu behandelnde Medium führende Bereich, d.h. der Reaktor selbst, zumindest teilweise als Reflektor ausgeführt, der Reflektor ist Teil des Reaktors. Die Ausbildung des Reaktors zumindest teilweise als Reflektor führt dazu, dass das Licht, das nach außen abgestrahlt werden würde, stattdessen wieder in den Reaktor hinein reflektiert wird. Ferner weist die Anordnung gemäß der DE 38 24 647 A1 durch die außen liegenden UV-Lichtquellen einen großen Bauraum auf.

Anordnungen dieser Art ermöglichen ein relativ homogenes Strahlungsfeld innerhalb des zu desinfizierenden Mediums. Nachteilig an diesen Anordnungen ist jedoch der große Bauraum der für die Strahlungsverteilung benötigt wird.

Systeme gemäß dem zweiten konzeptionellen Ansatz sind daher nicht für Anwendungen mit geringem Bauraum geeignet.

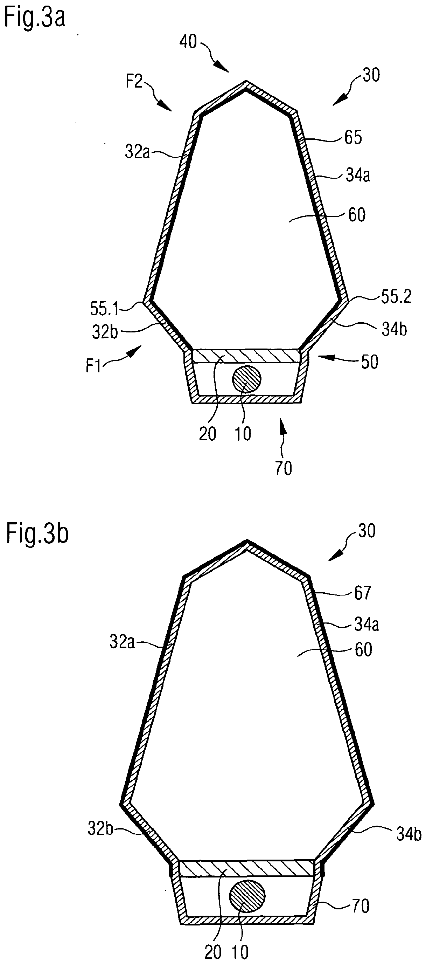

Die DE 10 2004 021 585 A1 offenbart eine Vorrichtung zur Entkeimung, umfassend ein Gehäuse, ein innerhalb oder unterhalb des Gehäuses angeordnetes Gebläse, welches Luft in das Gehäuse einsaugt und eine Luftströmung in axialer Richtung (Längsrichtung) durch das etwa rohrförmige Gehäuse, vorzugsweise von unten nach oben erzeugt, wobei innerhalb des Gehäuses Leuchtmittel angeordnet sind, die eine Ultraviolettstrahlung überwiegend im Bereich des keimabtötenden UV-Lichts erzeugen. Insbesondere zeigt Figur 3 einen Reaktor mit einem gleichmäßigen achteckigen Querschnitt.

Die EP 0 351 518 A2 bezieht sich auf eine Vorrichtung zur Bestrahlung von Flüssigkeiten und/oder Gasen mittels UV-Licht, bestehend aus einem von dem zu bestrahlend Medium durchströmten Rohrkörper mit mindestens zwei Stück außenachsparallel angeordneten UV-Lichtquellen mit Reflektoren.

Die WO 2007/027419 A2 bezieht sich auf eine Vorrichtung zur Behandlung einer Flüssigkeit, umfassend eine Kammer, wobei die Kammer mindestens 80 % umschlossen ist, sowie eine Ultraviolett-Lampe, die in der Kammer enthalten ist und eine Ultraviolett-durchlässige Röhre, die durch die Kammer verläuft, um Flüssigkeit hindurch passieren zu lassen, sowie eine reflektive Materialbeschichtung der Kammer, wobei das Material zu mindest 80 % reflektiv ist.

Die WO 2011/075694 A1 offenbart Vorrichtungen und Verfahren zur Behandlung von Flüssigkeiten mit UV-Licht enthaltend Fluidströme unter Verwendung von elliptischen Kammern. In den Kammern können Wasser oder andere Fluide desinfiziert werden.

Die US 2005/0115498 A1 beschreibt eine Vorrichtung zur Bereitstellung von Reflektoren für UV-härtende Systeme, wobei ein diffus reflektierendes Material verwendet wird und der von einer Oberfläche reflektierte Gesamtfluss auf die zu belichtende Oberfläche erhöht wird. Der Reflektor kann viele Formen einnehmen, einschließlich einer parabolischen oder kreisförmigen Form.

Die WO 2004/011915 A1 bezieht sich auf Vorrichtungen und Verfahren zum Behandeln eines Flüssigkeitsvolumens. Die Vorrichtung umfasst eine Fließpassage, durch die ein Fluss fließt, mindestens eine Bestrahlungsquelle, externe Passagen und mindestens zwei verlängerte elliptische reflektierende Öffnungen zum Reflektieren der Strahlung von mindestens einer Strahlungsquelle auf die Flüssigkeitspassage.

Schließlich beschreibt die WO 02/14925 A2 eine Lampenstruktur, umfassend elliptische Reflektoren für die gleichförmige Bestrahlung der Oberfläche eines Arbeitsstücks, wie einer optischen Faser, und ein Verfahren zur Verwendung dieser Lampenstruktur.

Aus dem Stand der Technik bekannte Anlagen weisen daher entweder eine kompakte Bauform, aber unzureichende Strahlungshomogenität auf; oder die aus dem Stand der Technik bekannten Anlagen erzielen zwar eine hohe Strahlungshomogenität, benötigen dafür aber einen großen Bauraum, der Anwendungen in beengten Einbausituationen ausschließt.

Demnach liegt der vorliegenden Erfindung die Aufgabe zugrunde, ein System bereitzustellen, welches die Nachteile des Standes der Technik vermeidet, d.h. eine ausreichend hohe Strahlungshomogenität bereitstellt und gleichzeitig eine hohe Kompaktheit der Bauweise ermöglicht.

Erfindungsgemäß wird die Aufgabe der vorliegenden Erfindung durch ein System zur Behandlung von Gasen und/oder Flüssigkeiten mit UV-Strahlung, umfassend zumindest eine UV-Lichtquelle sowie einen Reaktor, wobei der Querschnitt des Reaktors die Form eines unregelmäßigen Vielecks aufweist, der Seitenflächen, ein erstes die Seitenflächen verbindendes Teil oder Abschlussteil sowie einen zur Vorder- und Rückseite offenen Innenraum umfasst, durch den das Medium strömt, wobei der Reaktor zumindest teilweise in Form eines Reflektors ausgebildet ist, der vom optischen System ausgesandte Strahlung in den Innenraum des Reaktors reflektiert, wobei der Reaktor in zwei Funktionsbereiche unterteilt ist, einen ersten Funktionsbereich F1, welcher dem mindestens einen optischen System am nächsten liegt, und einen zweiten Funktionsbereich F2, welcher von dem mindestens einen optischen System weiter entfernt angeordnet ist als der erste Funktionsbereich F1, wobei sich im Betriebszustand des Systems Strahlung im ersten Funktionsbereich ungehindert ausbreiten kann und im zweiten Funktionsbereich Überlagerungen der Strahlung vorliegen und
a) im ersten Funktionsbereich F1 des Reaktors der Abstand zwischen den sich gegenüberliegenden Seitenflächen des Reaktors sich mit zunehmender Entfernung zu dem mindestens einen optischen System vergrößert,
wobei
der Querschnitt des Reaktors die Form eines unregelmäßigen Vielecks aufweist und wobei ein UV-transparenter Bereich vorgesehen ist, der gemäß Variante a) in Form eines UV-transparenten Fensters am Eintrittsteil des Reaktors vorgesehen ist.

Bevorzugt sind keine Aussparungen und/oder Einbuchtungen im Reaktor gemäß der DE 10 2011 112 994 A1 vorgesehen.

Das Medium-führende Bauteil in Form des Reaktors übernimmt damit gemäß der vorliegenden Erfindung gleichzeitig die Funktion eines Reflektors. Die Homogenisierung des Strahlungsfelds findet dabei durch Reflexionen an den Wänden des Reaktors statt und nicht - wie im Stand der Technik üblich - außerhalb desselben. Dadurch dass erfindungsgemäß der Reaktor selbst zumindest teilweise als Reflektor fungiert, gelingt es in der vorliegenden Erfindung in unerwarteter Weise die Homogenität der Strahlung, die vom optischen System ausgestrahlt wird oder zu diesem ausgestrahlt wird, zu verbessern, so dass ein deutlich effizienteres System bereitgestellt werden kann.

### Das optische System ist erfindungsgemäß eine UV-Lichtquelle

Da das(die) optische(n) System(e) eine (oder mehrere) UV-Lichtquelle(n) darstellt, liegt das erfindungsgemäße System in Form eines UV-Desinfektionssystems vor.

Im Folgenden wird das Funktionsprinzip der Verständlichkeit halber überwiegend anhand eines optischen Systems bestehend aus einer Lichtquelle beschrieben.

Unter "Reaktor" wird im Rahmen der vorliegenden Erfindung ein nicht notwendigerweise nach allen Seiten abgegrenzter Raum verstanden, der derart aufgebaut ist, dass in diesem unter definierten Bedingungen die Behandlung eines zu behandelnden Mediums, wie die UV-Desinfektion eines Mediums, beispielsweise Wasser, erfolgt.

Aussparungen und/oder Einbuchtungen im Reaktor vorzusehen, wie dies gemäß der DE 10 2011 112 994 A1 erfolgt, in denen sich das optische System befindet, um beispielsweise das im Innenraum strömende Medium zu bestrahlen, ist erfindungsgemäß nicht vorgesehen.

Die Zahl und Anordnung der optischen Systeme in Form von UV-Lichtquellen sind erfindungsgemäß zunächst nicht besonders beschränkt. Bevorzugt liegt nur ein optisches System vor. Es können aber auch 2 optische Systeme oder mehr vorhanden sein. Beispielhafte Ausführungsformen umfassen 1 bis 8 optische Systeme, bevorzugter 1 bis 6 optische Systeme, insbesondere 1 bis 5 optische Systeme, ganz besonders bevorzugt sind 1 bis 4 oder 1 bis 3 optische Systeme. Im Falle, dass als optische Systeme bspw. UV-LEDs eingesetzt werden, können erfindungsgemäß aber auch deutlich mehr optische Systeme vorhanden sein, beispielsweise 100 UV-LEDs oder mehr. Bevorzugt können mehrere optische Systeme nebeneinander angeordnet sein. Die Auswahl einer geeigneten Anordnung der optischen Systeme hängt neben der Anzahl, Größe, Form und Funktion der optischen Systeme auch von der gewählten Form und Größe des Reaktors sowie der gewählten Funktion, die das System erfüllen soll, ab. Ein Fachmann aus dem Stand der Technik kann ohne weiteres eine geeignete Anordnung der optischen Systeme für jeden Reaktor auswählen.

Das System kann mit außerhalb oder innerhalb des Reaktors liegendem/liegenden optischen System/en bereitgestellt werden. "Außerhalb des Reaktors" bedeutet, dass sich das (die) optische(n) System(e) nicht im Innenraum des Reaktors befindet(befinden), durch den das Medium strömt; "innerhalb des Reaktors" bedeutet, dass das(die) optische(n) System(e) sich im Innenraum des Reaktors befindet(befinden), wo das Medium hindurchströmt.

Der Reaktor ist erfindungsgemäß keine nach außen abgeschlossene Einheit, sondern bezeichnet einen zylinderförmigen Hohlkörper, der an beiden gegenüberliegenden Seiten, hier bezeichnet als Vorder- und Rückseite des Reaktors, offen ist, an einer Seite, der Vorderseite, strömt das Medium in den Reaktor hinein und an der anderen Seite, der Rückseite, strömt es wieder heraus. Der Reaktor ist bevorzugt ein Durchflussreaktor. Der zylinderförmige Hohlkörper weist zwei einander gegenüberliegende Seitenflächen definierter Wandstärke auf, die in einem ersten Teil und in einem weiteren Teil jeweils abgeschlossen sind, und einen Innenraum umgeben. Der Reaktor ist somit ein Hohlzylinder in Form eines geraden oder schiefen allgemeinen Zylinders. Ein Zylinder mit Grund- und Deckfläche entsteht durch Verschiebung einer ebenen Fläche oder Kurve entlang einer Geraden, die nicht in dieser Ebene liegt. Wenn die Geraden senkrecht zu Grund- und Deckfläche sind, spricht man von einem geraden Zylinder. Der erfindungsgemäße Reaktor in Form eines Hohlzylinders ist an beiden Seiten nicht durch eine Grundfläche und Deckfläche begrenzt, wie ein allgemeiner Zylinder, sondern offen ausgestaltet. Das zu verwendende oder zu behandelnde Medium strömt bspw. auf einer Seite, der Vorderseite, (quasi der weggelassenen Grundfläche eines allgemeinen Zylinders) in den Reaktor hinein und auf der anderen Seite, der Rückseite, (quasi der weggelassenen Deckfläche eines allgemeinen Zylinders) aus dem Reaktor hinaus. Während der Passage durch den Reaktor in Form des zylinderförmigen Hohlkörpers kann das Medium behandelt, bspw. desinfiziert oder erhitzt werden. Erfindungsgemäß bevorzugt leitet sich der Hohlzylinder von einem geraden allgemeinen Zylinder ab. Der nach vorne und hinten offene Hohlraum im Hohlzylinder bildet den Innenraum des Reaktors.

Der Querschnitt des Reaktors liegt in Form eines unregelmäßigen Vielecks vor. Die Form und Größe des Reaktors können im Rahmen der Erfindung beliebig ausgewählt werden, sofern die baulichen Gegebenheiten für die beabsichtigte Verwendung dies ermöglichen. Die Grenzen ergeben sich nur anhand der technischen Realisierbarkeit und der Handhabungseigenschaften.

An die Seitenflächen des Reaktors schließen sich auf beiden Seiten jeweils ein die beiden Seitenflächen verbindender Teil, wie beispielweise ein Eintrittsteil und ein Abschlussteil, unmittelbar an, wodurch der Reaktor gebildet wird. Durch diese ausgewählte Geometrie des Reaktors finden derartige Überlagerungen der Strahlengänge statt, dass eine Abschwächung der Strahlung durch Beiträge von den Wänden reflektierter Strahlen ausgeglichen wird. Somit bleibt die kumulierte Strahlungsintensität über den gesamten Reaktor weitestgehend unverändert. Hieraus resultiert eine besonders hohe Strahlungshomogenität über den gesamten Innenraum des Reaktors, wodurch eine verbesserte Behandlungs- oder Detektionsleistung erzielt wird. Ein derartiger Aufbau zeichnet sich zudem durch eine hohe Kompaktheit aus.

Erfindungsgemäß wird das Medium-führende Bauteil in Form des Reaktors in zwei Funktionsbereiche unterteilt. Dabei wird der Reaktor derart ausgestaltet, dass dieser aus einem ersten Funktionsbereich F1, welcher dem mindestens einen optischen System am nächsten kommt, und einem zweiten Funktionsbereich F2, welcher von dem mindestens einen optischen System weiter entfernt angeordnet ist, aufgebaut ist, wobei sich die vom oder zum optischen System ausgesandte Strahlung im ersten Funktionsbereich im Wesentlichen ungehindert ausbreitet und im zweiten Funktionsbereich im Wesentlichen Überlagerungen der Strahlung vorliegen und im ersten Funktionsbereich F1 im Reaktor der Abstand zwischen den sich gegenüberliegenden Seitenflächen des Reaktors sich mit zunehmender Entfernung zu dem mindestens einen optischen System vergrößert, bevorzugt kontinuierlich vergrößert (Variante a)). Erfindungsgemäß hat es sich gezeigt, dass zur Erlangung einer besonders homogenen Strahlungsintensität in den Systemen der vorliegenden Erfindung besonders vorteilhaft ist, wenn im ersten Funktionsbereich eine Vergrößerung des Innenraums des Reaktors (in Richtung zum Abschlussteil hin) vorgesehen ist.

Nach einer weiteren Ausgestaltung wird der Reaktor bevorzugt derart ausgestaltet, dass der Abstand zwischen den sich gegenüberliegenden Seitenflächen des Reaktors im zweiten Funktionsbereich F2 mit zunehmender Entfernung zu dem mindestens einen optischen System, vorzugsweise kontinuierlich, abnimmt. Erfindungsgemäß hat es sich ebenfalls gezeigt, dass zur Erlangung einer besonders homogenen Strahlungsintensität in den Systemen der vorliegenden Erfindung besonders vorteilhaft ist, wenn im zweiten Funktionsbereich eine Verjüngung des Innenraums des Reaktors (in Richtung zum Abschlussteil hin) vorgesehen ist.

Gemäß dieser Ausführungsform wird das mindestens eine optische System innerhalb des Reaktors vorgesehen.

Nach einer weiteren erfindungsgemäßen Variante wird der Reaktor bevorzugt derart ausgestaltet, dass der Abstand zwischen den sich gegenüberliegenden Seitenflächen des Reaktors im ersten Funktionsbereich F1 mit zunehmender Entfernung zu dem mindestens einen optischen System sich, vorzugsweise kontinuierlich, vergrößert und dass der Abstand zwischen den sich gegenüberliegenden Seitenflächen des Reaktors im zweiten Funktionsbereich F2 mit zunehmender Entfernung zu dem mindestens einen optischen System, vorzugsweise kontinuierlich, abnimmt.

Der erste Funktionsbereich (F1) kann sich hierbei im Innenraum und damit im Medium-führende Bereich des Reaktors befinden. Dies ist aber nicht notwendigerweise der Fall. Im ersten Funktionsbereich kann sich die von einer Strahlungsquelle, beispielsweise in Form des optischen Systems, aus gesehene Strahlung ungehindert ausbreiten. In diesem Bereich (Funktionsbereich 1) nimmt die Intensität der Strahlung aufgrund der räumlichen Ausbreitung sowie einer möglichen Absorption durch das strömende Medium mit zunehmendem Abstand von der Strahlungsquelle ab. Nach einer durch die Geometrie des Reaktors vordefinierten Wegstrecke trifft die Strahlung dann auf die reflektierenden Seitenflächen und wird unter einem Winkel zurückreflektiert. Dieser Winkel ist durch die Geometrie des Reaktors derart bestimmt, dass im zweiten Funktionsbereich im Wesentlichen eine Überlagerung der Strahlengänge stattfindet. Dadurch wird die Abschwächung der Strahlung durch Beiträge von den Wänden reflektierter Strahlen ausgeglichen, so dass die kumulierte Strahlungsintensität über den gesamten zweiten Funktionsbereich weitestgehend unverändert bleibt.

Der zweite Funktionsbereich (F2) ist somit der neben dem ersten Funktionsbereich verbleibende Bereich im Reaktor, bei dem sich von einer Strahlungsquelle aus gesehen der Innenraum des Reaktors in Richtung des Abschlussteils bevorzugt verjüngt. Dies kann beispielsweise durch eine Neigung der Seitenflächen nach Innen, d.h. in entgegen gesetzter Richtung, um jeweils einen entsprechenden Winkel (kleiner als 90° zu einer horizontalen Ebene durch den Reaktor) erfolgen.

Die genaue Form des Medium-führenden Bauteils in Form des Reaktors hängt dabei von der Stärke der Strahlungs-Absorption des Mediums, den Reflexionseigenschaften der Seitenflächen, der zu erzielenden Mindeststrahlungsdichte und eventuellen Bauraumbeschränkungen ab.

Erfindungsgemäß wird der erste Funktionsbereich F1 derart ausgewählt, dass sich der Reaktor mit steigender Entfernung zu dem mindestens einen optischen System zunehmend erweitert, d.h. der Abstand der Seitenflächen nimmt mit steigender Entfernung zu dem mindestens einen optischen System, vorzugsweise kontinuierlich, zu (Variante a)). "Kontinuierlich" bedeutet in diesem Zusammenhang, dass keine Unterbrechung der Seitenfläche vorliegt.

Entsprechend der oben erläuterten Variationen können die Funktionsbereiche demnach unterschiedlich ausgestaltet sein.

Erfindungsgemäß ist es weiterhin bevorzugt, wenn der Abstand zwischen den Seitenflächen im ersten Funktionsbereich mit steigender Entfernung zu dem mindestens einen optischen System, vorzugsweise kontinuierlich, zunimmt und im zweiten Funktionsbereich mit steigender Entfernung zu dem mindestens einen optischen System, vorzugsweise kontinuierlich, abnimmt. Der erste Funktionsbereich kann sich dann beispielsweise durch Vorsehen eines baulichen Übergangs an den zweiten Funktionsbereich anschließen. Dieser Übergang kann beispielsweise eine eckige Form, wie eine Ecke bzw. Kante, die jeweils in beiden Seitenflächen vorgesehen ist, darstellen.

Die die Seitenflächen verbindenden Teile, insbesondere in Form von Eintrittsteil und Abschlussteil, des Reaktors sind hinsichtlich Form und Größe relativ beliebig auswählbar. Diese dienen nur dazu den Strahlungsraum des Reaktors nach außen abzuschließen, d.h. die Seitenflächen miteinander zu verbinden, wodurch eine vorzugsweise in sich geschlossene Mantelfläche des Hohlzylinders resultiert. Vorzugsweise ist insbesondere das Abschlussteil des Reaktors ebenfalls in Form eines Reflektors ausgeführt, wodurch dieses zusätzlich zur Homogenität des Strahlungsfeldes beiträgt.

Das erfindungsgemäße Prinzip, wonach sich im Reaktor die Strahlung in einem ersten Funktionsbereich zunächst ausbreitet und dann im zweiten Funktionsbereich eine Überlagerung der Strahlengänge stattfindet, kann sowohl für Systeme mit außen liegenden als auch innen liegenden optischen Systemen, d.h. UV-Lichtquellen zum Einsatz kommen.

Das/die optische(n) System(e) befindet (befinden) sich außerhalb des Reaktors, wobei ein vordefinierter Strahlungs-transparenter Bereich in Form eines Strahlungs-transparenten Fensters im Reaktor vorgesehen ist. Dieses Strahlungs-transparente Fenster ist im Eintrittsteil des Reaktors vorgesehen oder bildet den Eintrittsteil des Reaktors aus. Durch dieses Strahlungs-transparente Fenster gelangt die Strahlung entweder von einem oder mehreren optischen Systemen in Form von ein oder mehreren UV-Lichtquellen, die außerhalb des Reaktors angeordnet sind, in den Innenraum des Reaktors, der in einen ersten und zweiten Funktionsbereich aufgeteilt ist. Besonders bevorzugt verbindet das Strahlungs-transparente Fenster daher die beiden Seitenflächen des Reaktors im oder als Eintrittsteil.

Der zweite Funktionsbereich F2 beginnt dort, wo sich der erste Funktionsbereich in einen oder mehrere sich anschließende verjüngende Bereiche übergeht.

Die Verjüngung des zweiten Funktionsbereichs F2 im Reaktor trägt daher erfindungsgemäß in hohem Maße zur Homogenisierung der vorhandenen Strahlung bei.

Die mit den erfindungsgemäßen Systemen, in Form der Variante a) erhaltene hohe Strahlungshomogenität kann anhand der bereits geschilderten Ray Tracing-Methode quantifiziert werden. So liegt die Standardabweichung vom Mittelwert der Strahlungsdichte im Reaktor erfindungsgemäß bei < 30%, bevorzugt < 25%, bevorzugter < 20%, noch bevorzugter < 15%, insbesondere ≤ 13%, ganz besonders bevorzugt ≤ 10%. Erfindungsgemäß werden in der Regel Werte im Bereich von 10 bis 20% erzielt.

Die Anordnungen aus dem Stand der Technik zeigen demgegenüber teilweise Werte von über 40%, so dass die erfindungsgemäßen Systeme diesen Anordnungen im Hinblick auf die Homogenität überlegen sind.

Zudem werden erfindungsgemäß besonders kompakte Systeme zur Verfügung gestellt. Dies kann beispielsweise durch den Anteil des Volumens des verwendeten/zu behandelnden Mediums am Gesamtvolumen des Systems ausgedrückt werden. Der Anteil des im Reaktor vorhandenen oder zu behandelnden Mediums entspricht in der Regel dem Innenvolumen des Reaktors. Es gibt aber auch Ausführungsformen, in denen dies nicht so ist, wenn beispielsweise ein Teil des Innenraums nicht mit Medium gefüllt oder von diesem durchströmt wird. In der vorliegenden Erfindung ist der Volumenanteil des Mediums am Gesamtvolumen sehr groß, d.h. es ist neben dem im Reaktor vorhandenen Volumen des verwendeten/zu behandelnden Mediums kaum zusätzlicher und damit überflüssiger Raum im System vorhanden. Als Faustregel kann man angeben, dass der Anteil des Volumens des verwendeten/zu behandelnden Mediums oder der Anteil des Volumens des Innenraums am Gesamtvolumen des Systems erfindungsgemäß bevorzugt mindestens etwa 60%, bevorzugter mindestens etwa 70%, insbesondere mindestens etwa 80%, ganz besonders bevorzugt mindestens etwa 90% beträgt. Die erläuterten Anordnungen aus dem Stand der Technik bieten demgegenüber einen Anteil des Volumens des verwendeten/zu behandelnden Mediums am Gesamtvolumen des Systems der im Bereich von 10 bis 20% beträgt, wie jeweils aus Figur 1 der GB 2 334 873 A und Figur 1 der US 5 247 178 A hervorgeht.

Für den Reaktor, der aus erstem und zweitem Funktionsbereichen aufgebaut ist, werden für die Gesamtform unregelmäßige Vielecke, ausgewählt, die in vielfacher Hinsicht variiert werden können.

Die Wandstärke des Reaktors kann erfindungsgemäß zunächst beliebig eingestellt werden. Beschränkungen liegen nur im Hinblick auf den geplanten Einsatzzweck, die gewünschte Form und Größe sowie die gewünschten mechanischen Stabilitätsanforderungen vor.

Für eine UV-Desinfektion ist zu berücksichtigen, dass das zu desinfizierende Medium, insbesondere Wasser, häufig unter Druck steht. Beispielsweise liegen im Haushaltsbereich Außenanschlussdrücke von 4 bis 8 bar vor, die aber im weiteren Verlauf, z.B. bei Auslauf eines Wasserhahns, deutlich abfallen können, z.B. auf <1 bar. In der großtechnischen Wasseraufbereitung liegen die Drücke oft wesentlich höher, so dass der Reaktor - je nach Einsatzzweck und Einsatzort - für bestimmte Drücke ausgelegt sein sollte. Dies stellt jedoch Wissen des Fachmanns dar, der ohne weiteres die geeignete Wandstärke für einen Reaktor für das jeweilige Anwendungsgebiet auswählen kann.

Die genaue Geometrie des Reaktors, insbesondere Maße, Winkel und dergleichen, kann daher in Abhängigkeit der Anzahl, Anordnung und Form der optischen Systeme, dem Strahlungs-Absorptionsgrad und der Art des verwendeten Mediums, den Reflektionsverlusten an den Reflektor-Spiegelflächen, sowie weiteren Verlustmechanismen bestimmt und ausgewählt werden. Diese sind daher auf den spezifischen Anwendungsfall anzupassen. Ein Fachmann kann dies ohne weiteres anhand der vorliegenden Beschreibung, seiner Fachkenntnis sowie entsprechender Literatur durchführen.

Die Form und Größe des Reaktors bestimmt daher zumindest teilweise die Form und Größe des Reflektors, je nach Ausführungsvariante. Den Reaktor erfindungsgemäß zumindest teilweise als Reflektor auszubilden, kann auf verschiedene Art und Weise erfolgen:

In einer erfindungsgemäßen Ausführungsform kann die gesamte Mantelfläche des Reaktors selbst, d.h. die beiden Seitenflächen, das Eintrittsteil und das Abschlussteil des Reaktors, oder Teile hiervon als Reflektor ausgeführt sein. Bevorzugt wird nur eine Fläche oder Teilfläche des Reaktors nicht als Reflektor ausgeführt. Besonders bevorzugt wird der Reaktor erfindungsgemäß bis auf das Eintrittsteil des Reaktors als Reflektor ausgeführt.

Erfindungsgemäß liegt ein vordefinierter Strahlungs-transparenter Bereich oder ein Strahlungs-transparentes Fenster im Reaktor vor der/das dazu dient, das Strahlung aus einem oder mehreren optischen Systemen, bspw. in Form von einer oder mehreren UV-Lichtquellen hindurchtreten kann. Form und Größe des Strahlungs-transparenten Bereichs/Fensters können je nach Anzahl, Größe und Form des verwendeten optischen Systems ausgewählt und angepasst sein, so dass ein entsprechend großer "Öffnungswinkel" für das(die) optische(n) System(e) zur Verfügung steht. Es können auch mehrere Strahlungs-transparente Bereiche oder Fenster im Reaktor vorgesehen sein. Besonders bevorzugt liegt nur 1 Strahlungs-transparentes Fenster vor. Bevorzugt wird für jedes optische System oder eine Gruppe von optischen Systemen jeweils ein Strahlungs-transparenter Bereich im Reaktor vorgesehen. Es liegt ein Strahlungs-transparentes Fenster im Eintrittsteil des Reaktors vor oder bildet das Eintrittsteil aus, so dass der Innenraum des Reaktors von dem mindestens einen optischen System und einem gegebenenfalls vorhandenen Reflektor getrennt ist. Das Strahlungs-transparente Fenster dient dazu, die Strahlung von ein oder mehreren optischen Systemen, die außerhalb des Reaktors angeordnet sind, in den Innenraum des Reaktors durchzulassen oder die Strahlung aus dem Innenraum des Reaktors zu ein oder mehreren optischen Systemen durchzulassen.

Das Material, aus dem der Reflektor besteht, ist nicht besonders beschränkt, es kann jedes Material oder jede Kombination von Materialien verwendet werden, das/die der Fachmann für einen Reflektor verwenden würde und hierfür kennt. Der Reflektor kann z.B. aus flexiblem oder starrem bzw. festem Material aufgebaut sein. Beispielsweise kann die Wand des Reaktors, je nach Ausführungsform teilweise oder vollständig, aus einem Material oder einer Materialkombination aufgebaut sein, das/die das Licht der gewählten Lichtquelle reflektiert. Ein beispielhaftes Material ist Aluminium.

Nach einer weiteren erfindungsgemäßen Variante kann der Reflektor auf die Wand des Reaktors in Form einer UV-Strahlungs-reflektierenden Außen- oder Innenschicht oder -beschichtung aufgebracht sein. Beispielsweise kann eine Strahlungs-reflektierende Schicht oder Beschichtung auf der Innenseite der Wand des Reaktors aufgebracht sein. In diesem Fall wird der Reflektor direkt auf die Innenwand des Reaktors aufgebracht oder auf die Innenseite beschichtet. Das Material aus dem der Reflektor besteht, ist nicht weiter beschränkt, sofern dieses für den Einsatzzweck als geeignet anzusehen ist. Die Strahlungs-reflektierende Schicht oder Beschichtung kann aus einer Vielzahl von Materialien oder Materialkombination ausgewählt sein. Beispielsweise kann auch ein Mehrschichtsystem zum Einsatz kommen. Der Reflektor kann z.B. aus einem kostengünstigen Metall oder einer kostengünstigen Metalllegierung gefertigt sein. Andere Materialien sind ebenfalls möglich. Der Vorteil einer Strahlungs-reflektierenden Innenschicht oder Innenbeschichtung ist, dass das reflektierte Licht nicht wie bei einer Außenschicht oder -beschichtung durch die Passage durch die Wand zum Reflektor hin durch Restabsorption abgeschwächt wird.

Die Strahlungs-reflektierende Innenschicht oder -beschichtung kann zusätzlich durch eine Schutzschicht vor dem zu desinfizierenden Medium geschützt werden. Dies ist jedoch nicht in jedem Fall erforderlich. Wenn z.B. Wasser das verwendete Medium darstellt, kann ein wasserbeständiges Material, das UV-transparent ist, als Schutzschicht oder -beschichtung eingesetzt werden.

Der Reflektor kann auch eine Strahlungs-reflektierende Schicht oder Beschichtung auf der Außenseite des Reaktors darstellen. In diesem Fall wird der Reflektor direkt auf die Außenwand des Reaktors aufgebracht oder auf die Außenseite beschichtet. Bei Vorsehen einer Außenschicht oder Beschichtung ist der Reaktor selbst aus Strahlungs-transparentem Material, z.B. UV-transparentem Glas, aufgebaut. Eine derartige Strahlungs-reflektierende Schicht oder Beschichtung kann aus einem Material oder einer Materialkombination aufgebaut sein. Beispielsweise kann auch ein Mehrschichtsystem vorliegen.

Der Begriff "Strahlungs-transparent" bedeutet, dass das erfindungsgemäß eingesetzte Material eine hohe Transmission für bestimmte Strahlung aufweist, was bedeutet, dass eine Transmission von mindestens 75% bei einer entsprechenden Wellenlänge der eingesetzten Strahlung (z.B. einer Wellenlänge von 254 nm bei UV-Strahlung) oder in einem entsprechenden Wellenlängenbereich und einer Schichtdicke des Materials von 1 mm vorliegt.

Nach einer besonders bevorzugten Ausführungsform zeigt das Material bei UV-Strahlung eine Transmission bei einer Schichtdicke von 1 mm im UV-Bereich, die bei 200 nm < 5% liegt und bei 254 nm > 75% liegt. Noch bevorzugter wird eine Transmission bei einer Schichtdicke von 1 mm im UV-Bereich bei 200 nm < 1% und bei 254 nm > 80% erhalten. Besonders bevorzugtes UV-transparentes Material ist beispielsweise UV-transparentes Glas, z.B. Quarzglas.

Es versteht sich, dass das Material des erfindungsgemäßen Systems entsprechend dem ausgewählten optischen System, beispielsweise entsprechend der Strahlungswellenlänge der verwendeten Lichtquelle(n), ausgewählt wird, um je nach Ausführungsform die entsprechende Strahlung durchzulassen oder zu reflektieren und von der Strahlung nicht angegriffen und verändert wird. Die jeweils geeigneten Materialien sind dem Fachmann im Stand der Technik ohne weiteres bekannt und können von diesem ausgewählt werden.

Zusätzlich zu dem zumindest teilweise als Reflektor ausgebildeten Reaktor können ein oder mehrere einzelne Reflektoren vorgesehen werden, die vorzugsweise hinter der oder den vorhandenen optischen Systemen angeordnet sind. Diese zusätzlichen Reflektoren werden nachfolgend auch als Lampenreflektoren oder zweite Reflektoren bezeichnet. Diese Ausführungsform kommt insbesondere dann zum Einsatz, wenn das(die) optische(n) System(e) außerhalb des Reaktors angeordnet ist(sind). Bevorzugt ist jedem optischen System oder jeder Gruppe von optischen Systemen ein Reflektor zugeordnet, um eine möglichst hohe Strahlungsenergie für das im Reaktor fließende oder strömende oder vorliegende Medium zur Verfügung zu stellen. Insbesondere bei einem ungerichteten optischen System ist das Vorsehen von einem oder mehreren Reflektoren bevorzugt. Der Reflektor hinter dem optischen System hat die Funktion, das in die falsche Richtung abgestrahlte Licht in den Reaktor zu reflektieren.

Die einzelnen den jeweiligen optischen Systemen zugeordneten Reflektoren sind in beliebiger Form wählbar, verschiedenste Reflektorgeometrien eignen sich hierfür. Eine optimale Strahlungshomogenität ergibt sich, wenn der erste und zweite Reflektor aufeinander abgestimmt sind. Vorzugsweise weist der dem optischen System zugeordnete Reflektor eine runde Form, wie einen Hohlspiegel in Form eines Kugelausschnitts, oder eine eckige Form auf. Bevorzugt umschließt der jedem optischen System zugeordnete Reflektor diese dabei derart, dass die vom optischen System, beispielsweise in Form einer Lichtquelle, ausgesandte Strahlung nur in Richtung des Reaktors abgestrahlt wird. Die Reflektoren können daher in einer beliebigen geometrischen Form ausgewählt werden, wobei diese zu einer Seite hin offen ausgestaltet ist, damit das Licht vom optischen System im wesentlichen in eine Vorzugsrichtung abgestrahlt werden kann.

Nach einer weiteren bevorzugten Ausführungsform können der Reaktor, der teilweise als Reflektor (erster Reflektor) ausgebildet ist, und der einem optischen System zugeordnete Reflektor (zweiter Reflektor) derart aufgebaut und angeordnet sein, dass diese in Kontakt miteinander stehen oder einander derart überlappen, so dass eine Art Gesamtreflektor mit einem gemeinsamen Strahlungsraum ausgebildet wird. Es ist hierbei zu beachten, dass dieser Strahlungsraum im eigentlichen Sinne keinen abgeschlossenen Raum darstellt, sondern auf beiden Seiten offen ist, damit das vorhandene Medium hinein und wieder herausströmen kann. Durch diese erfindungsgemäße Ausgestaltung kann eine besonders hohe Homogenität der Strahlungsverteilung erreicht werden und es wird zudem eine besonders hohe Kompaktheit des erfindungsgemäßen Systems erhalten.

Der zweite Reflektor befindet sich vorzugsweise in Kontakt mit dem Reaktor, der zumindest teilweise als Reflektor ausgebildet ist. Der zweite Reflektor führt dadurch zusätzlich Wärme durch direkten Kontakt mit dem Reaktor ab.

Eine besonders bevorzugte Ausführungsform des Systems der vorliegenden Erfindung stellt der sogenannte "modifizierte Eingangstrichter" dar:

In dieser erfindungsgemäß bevorzugten Ausführungsform ist bevorzugt nur ein optisches System vorgesehen. Es können aber auch mehrere optische Systeme vorhanden sein. Das optische System ist außerhalb des Reaktors angeordnet.

Die Seitenflächen und das Abschlussteil des Reaktors sind als Reflektor (erster Reflektor) ausgebildet. Den Eintrittsteil des Reaktors bildet vorzugsweise ein Strahlungs-transparentes Fenster, das die beiden Seitenflächen miteinander verbindet. In einer Ausführungsform ist das optische System eine Lichtquelle, d.h. eine UV-Lichtquelle, und strahlt ihr Licht durch dieses Strahlungs-transparente Fenster in den Innenraum des Reaktors ab.

Das Abschlussteil des Reaktors wird bevorzugt durch eine eckige Form ausgebildet, die die beiden Seitenflächen miteinander verbindet. Besonders bevorzugt läuft der Reaktor im Abschlussteil spitz zu.

Besonders bevorzugt wird das optische System zusätzlich von einem Reflektor (Lampenreflektor oder zweiter Reflektor) derart umgeben, dass bspw. in die falsche Richtung abgestrahltes Licht in den Reaktor reflektiert wird.

Vorzugsweise sind der erste und zweite Reflektor dabei derart ausgebildet, dass diese einen Gesamtreflektor ausbilden. Dies kann beispielsweise dadurch erfolgen, dass sich der erste Reflektor und der zweite Reflektor in Kontakt befinden oder überlappend angeordnet sind.

Der Reaktor ist derart ausgestaltet, dass dieser aus einem ersten Funktionsbereich F1, welcher dem mindestens einen optischen System am nächsten kommt, und einem zweiten Funktionsbereich F2, welcher von dem mindestens einen optischen System weiter entfernt angeordnet ist, aufgebaut ist, wobei sich die vom oder zum optischen System ausgesandte Strahlung im ersten Funktionsbereich im Wesentlichen ungehindert ausbreitet und im zweiten Funktionsbereich im Wesentlichen Überlagerungen der Strahlung vorliegen. Hierbei wird gemäß Variante a) der erste Funktionsbereich so ausgestaltet, dass der Abstand zwischen den sich gegenüberliegenden Seitenflächen des Reaktors mit zunehmender Entfernung zum optischen System, vorzugsweise kontinuierlich, zunimmt. Der zweite Funktionsbereich wird vorzugsweise so ausgestaltet, dass der Abstand zwischen den sich gegenüberliegenden Seitenflächen des Reaktors mit zunehmender Entfernung zum optischen System, vorzugsweise kontinuierlich, abnimmt. Es kann ein baulicher Übergang vorliegen, bei dem der erste in den zweiten Funktionsbereich unmittelbar übergeht. Dies stellt hier eine Ecke bzw. Kante, jeweils in beiden Seitenflächen dar, deren Spitze jeweils nach außen gerichtet ist. Es kann aber auch ein kontinuierlicher Übergang zwischen dem ersten und zweiten Funktionsbereich vorliegen.

Im erläuterten modifizierten Eingangstrichter liegen im Innenraum des Reaktors zwei Funktionsbereiche vor, so dass der gesamte Innenraum des Reaktors über eine außerordentlich homogene Strahlungsdichte verfügt. Es liegen keine lokal stark reduzierten Strahlungsintensitäten vor, die zu einer unzureichenden Verteilung der Strahlungsleistung führen könnten.

Besonders bevorzugte Ausführungsformen des erfindungsgemäßen Systems sind bei der Figurenbeschreibung im Einzelnen erläutert.

Wenn das(die) optische(n) System(e) in Form von Lichtquellen ausgewählt wird(werden), so können prinzipiell beliebige Lichtquellen zum Einsatz kommen, es kann beispielsweise jede Art an bekannter UV- Lichtquelle Verwendung finden.

Bei UV-Strahlung wird üblicherweise eine Wellenlänge von 253,7 nm eingesetzt. Dies stellt die Hauptemissionswellenlänge von Niederdruck-UV-Lampen und ein wesentliches Strahlungsmaximum anderer UV-Lampen dar. Als UV-Lichtquellen eingesetzt werden daher beispielsweise Mitteldruck-, Hochdruck- oder Niederdruck-UV-Lampen, bevorzugt Quecksilberdampf-Mitteldruck-, -Hochdruck- oder -Niederdruck-Lampen, welche Strahlung bei einer Wellenlänge um 254 nm emittieren. Niederdruck-UV-Lampen, insbesondere Niederdruck-Quecksilberdampflampen sind besonders bevorzugt. Nach einer weiteren Ausführungsform der Erfindung sind UV-Lichtquellen in Form von CCL (Cold Cathode Lamp) besonders bevorzugt. Diese basieren auf der bewährten CCFL-Technologie (Cold Cathode Fluorescent Lamp), wobei auf die Fluoreszenz-Beschichtung verzichtet wird; diese können heute bereits frei am Markt bezogen werden. Erfindungsgemäß können auch UV-LEDs zum Einsatz kommen. Beim Einsatz von UV-LEDs kann eine höhere Wellenlänge im Bereich von 270 nm gewählt werden, bei der einerseits die Desinfektionswirkung größer ist; andererseits besitzen typische UV-transparente Gläser bei diesen Wellenlängen eine höhere Transmission, was die Effizienz zusätzlich erhöht.

Für das optische System können auch Lichtquellen eingesetzt werden, deren Strahlung vorzugsweise innerhalb eines definierten Austrittswinkels abgegeben wird, wie UV-LEDs, die beispielsweise außerhalb des Reaktors vorliegen können. Der Austrittswinkel bestimmt dann maßgeblich die Auswahl der Größe des Bereichs des Reaktors, der Strahlungs-transparent ausgestaltet wird, beispielsweise kann ein Strahlungs-transparentes Fenster entsprechender Breite und Länge vorgesehen sein. Gemäß einer bevorzugten Ausführungsform können die optischen Systeme in Form von Lichtquellen auch auf einer gemeinsamen Trägerplatte oder auch Leiterplatte befestigt werden, so dass sich die Beleuchtungseinheit kostengünstig herstellen, leicht montieren und wieder austauschen lässt. Eine separate Montage vieler einzelner optischer Systeme in Form von UV-Lichtquellen für den Reaktor entfällt somit.

Wenn als optische Systeme relativ stark strahlende Lichtquellen, wie übliche UV-Stablampen, zum Einsatz kommen, ist es aus Kostengründen bevorzugt, eine möglichst geringe Anzahl an optischen Systemen bzw. Lichtquellen einzusetzen, bevorzugt 1 bis maximal 3. Wenn als optische Systeme relativ schwach strahlende Lampen zum Einsatz kommen, wie UV-LEDs, so kann erfindungsgemäß auch eine deutlich größere Anzahl an Lichtquellen eingesetzt werden, beispielsweise 100 LEDs oder mehr. In jedem Fall ist es zweckmäßig, eine vordefinierte Mindeststrahlungsintensität nicht zu unterschreiten, um eine ausreichende Strahlung zu gewährleisten. Dies hängt jedoch vom jeweiligen Anwendungsfall ab.

Besonders bevorzugt wird das optische System oder werden die optischen Systeme parallel zur Strömungsrichtung des zu verwendenden Mediums innerhalb oder außerhalb des Reaktors angeordnet. Beispielsweise kann als optisches System eine UV-Stablampe als einzige UV-Lichtquelle eingesetzt werden, die vorzugsweise parallel zur Strömungsrichtung eines zu desinfizierenden Mediums, wie Wasser, innerhalb des Reaktors oder außerhalb des Reaktors vorgesehen ist.

Wenn im erfindungsgemäßen System ein Strahlungs-transparentes Material zum Einsatz kommt, so ist dies bevorzugt Strahlungs-transparentes Glas. Das verwendbare Strahlungs-transparente Glas ist im Rahmen der vorliegenden Erfindung nicht besonders beschränkt. Es kann jedes dem Fachmann bekannte Glas zum Einsatz kommen, das für die verwendete Strahlung entsprechend transparent ist.

Erfindungsgemäß bevorzugte UV-transparente Gläser sind beispielsweise Quarzgläser, Silicatgläser, besonders bevorzugt Borosilicatgläser oder Natrium-Kalium-Barium-Silicatgläser, ganz besonders bevorzugt Quarzgläser und Borosilicatgläser. Besonders bevorzugte Gläser sind in der DE 10 2011 112 994 A1 beschrieben, deren Offenbarung hierdurch Bezugnahme in vollen Umfang in die vorliegende Beschreibung aufgenommen wird.

Das zu verwendende Medium ist erfindungsgemäß nicht besonders beschränkt. Es kann jede Flüssigkeit oder jedes Gas oder auch eine Mischung von mehreren Flüssigkeiten oder Gasen oder eine flüssige oder gasförmige Lösung, Dispersion oder dergleichen, insbesondere auch eine Mischung aus zwei oder mehr Bestandteilen, im erfindungsgemäßen System verwendet werden. Ein bevorzugtes Medium ist Wasser. Nach einer bevorzugten Ausführungsform kann auch ein Gas desinfiziert werden; es kann zweckmäßig sein, wenn dies nicht Luft ist. Wenn besonders aggressive Gase oder Flüssigkeiten eingesetzt werden sollen, kann eine entsprechende Auswahl aus geeigneten Materialzusammensetzungen getroffen werden.

Gegenstand der Erfindung ist auch die Verwendung des erfindungsgemäßen Systems
- als UV-Desinfektionssystem:
   wobei das optische System mindestens eine UV-Lichtquelle umfasst, insbesondere zur Desinfektion von Flüssigkeiten und/oder Gasen in ruhendem oder strömendem Zustand, insbesondere zur Trinkwasseraufbereitung und -Desinfektion, Desinfektion von Reinstwasser, Abwasser, Flüssigkeiten aus dem pharmazeutischen und Lebensmittel-Bereich, zur Desinfektion von Gasen, wie Luft oder Industriegasen.

Die Vorteile der Erfindung sind außerordentlich vielschichtig:

Erfindungsgemäß wird ein System zur Behandlung von Gasen und/oder Flüssigkeiten mit UV-Strahlung bereitgestellt, wobei ein Reaktor zumindest teilweise in Form eines Reflektors ausgebildet ist, der die von ein oder mehreren optischen Systemen oder für ein oder mehrere optische Systeme bereitgestellte Strahlung in den

Innenraum des Reaktors reflektiert. Das Medium-führende Bauteil in Form des Reaktors übernimmt daher gleichzeitig die Funktion des Reflektors.

Hierdurch gelingt es die Kompaktheit des bereitgestellten Systems zu verbessern, da auf räumlich anspruchsvolle Reflektorgeometrien verzichtet werden kann. Lediglich zusätzliche dem(den) optischen System(en) zugeordnete zweite Reflektoren können Verwendung finden.

Durch Aufteilen des Innenraum des Reaktors in einen ersten und zweiten Funktionsbereich kann, wie beschrieben, erreicht werden, dass die Abschwächung der Strahlung mit zunehmendem Abstand von einem optischen System durch von den Wänden reflektierte Strahlen ausgeglichen werden, so dass die kumulierte Strahlungsintensität über den gesamten Funktionsbereich F1 und F2 weitestgehend unverändert bleibt, wodurch wieder eine besonders hohe Strahlungshomogenität über den gesamten Reaktor erzielt wird.

Durch Ausgestaltung des Reaktors, indem im ersten Funktionsbereich F1 der Abstand zwischen den sich gegenüberliegenden Seitenflächen des Reaktors mit zunehmender Entfernung zu dem mindestens einen optischen System, vorzugsweise kontinuierlich, zunimmt, gelingt es eine besonders hohe Strahlungshomogenität über den gesamten Innenraum zu erzielen.

Auch das zusätzliche Vorsehen einer Verjüngung in Richtung zum Abschlussteil des Reaktors hin in einem oder mehreren zweiten Funktionsbereichen trägt in hohem Maße zu einer Homogenisierung der Strahlung, d.h. UV-Strahlung, bei.

Erfindungsgemäß konnte anhand der Ray Tracing-Methode festgestellt werden, dass die Standardabweichung vom Mittelwert der Strahlungsdichte erfindungsgemäß < 30%, bevorzugt < 25%, bevorzugter < 20%, noch bevorzugter < 15% beträgt, insbesondere ≤ 13%, ganz besonders bevorzugt ≤ 10% liegt.

Anordnungen aus dem Stand der Technik zeigen deutlich größere und damit schlechtere Werte im Bereich von 40% oder mehr.

Zudem werden erfindungsgemäß besonders kompakte Systeme zur Verfügung gestellt. Der Anteil des Volumens des verwendeten/behandelten, beispielsweise desinfizierten oder erhitzen, Mediums oder der Anteil des Volumens des Innenraums am Gesamtvolumen des Systems liegt erfindungsgemäß bevorzugt bei mindestens etwa 60%, bevorzugter mindestens etwa 70%, insbesondere mindestens etwa 80%, ganz besonders bevorzugt mindestens etwa 90%. Anordnungen mit hoher Strahlungshomogenität aus dem Stand der Technik bieten demgegenüber einen Anteil des Volumens des verwendeten/behandelten Mediums am Gesamtvolumen des Systems, der im Bereich von 10 bis 20% liegt.

Das erfindungsgemäße System ermöglicht somit ein homogeneres Strahlungsfeld innerhalb des verwendeten Mediums, wobei anders als im Stand der Technik kein großer Bauraum für die Strahlungsverteilung benötigt wird. Das erfindungsgemäße System ist trotzdem relativ einfach aufgebaut und vermeidet überflüssigen Bauraum. Die Kombination aus hoher Homogenität der Strahlung sowie großer Kompaktheit des Systems führt zu effektiven Ergebnissen, wie wirksamerer Desinfektionswirkung bei Verwendung von UV-Strahlung bei der vorliegenden Erfindung.

Das erfindungsgemäße System ist zudem außerordentlich variabel auswählbar. Die optischen Systeme können sich außerhalb des Reaktors befinden. Das System kann gezielt an einen Anwendungsfall angepasst werden.

Dadurch dass der Reaktor zumindest teilweise als Reflektor fungiert und gegebenenfalls zusammen mit dem/den Reflektoren für jedes optische System einen Gesamtreflektor bilden kann, wird die zur Verfügung gestellte Strahlung optimal genutzt.

Je nach Auswahl des(der) optischen Systems(e) kann die Funktionsweise des erfindungsgemäßen Systems ausgewählt werden.

Als optische Systeme werden UV-Lichtquellen ausgewählt, so dass ein UV-Desinfektionssystem erhalten wird, das aufgrund der Homogenität des erzeugten Strahlungsfelds einen besonders hohen Wirkungsgrad aufweist und durch die kompakte Ausführung auch bei vorgegebenen starken räumlichen Beschränkungen zum Einsatz kommen kann.

Es versteht sich, dass die verwendeten Materialien auf die UV-Strahlung abgestimmt und ausgewählt werden sollten, um je nach Ausführungsform die entsprechende Strahlung durchzulassen oder zu reflektieren und von der Strahlung nicht angegriffen und verändert zu werden.

Von großem Vorteil ist auch, dass das erfindungsgemäße System ohne Einwirkung von außen funktioniert. Das erfindungsgemäße System kann in einem kompakten Gehäuse untergebracht werden. Das System kann problemlos in größeren Einheiten, beispielsweise mit fließendem Medium, wie einem Rohrsystem, oder auch mit ruhendem Medium, wie einem Tank oder dergleichen, eingesetzt werden. Das System kann stationär, fest eingebaut, als Teil eines größeren Systems oder flexibel handhabbar als Handgerät zum Einsatz kommen.

Die erfindungsgemäße Vorrichtung erzielt damit einen möglichst hohen Wirkungsgrad bei relativ geringem Kostenaufwand bei der Herstellung.

Die erfindungsgemäße Vorrichtung ist auch für sehr spezielle Anforderungen geeignet. Beispielsweise als UV-Desinfektionssystem für die Herstellung von hochreinem Wasser, das insbesondere im Bereich der Pharma-, Kosmetik- und Halbleiterindustrie benötigt wird.

Das System der Erfindung zeigt seine Vorteile insbesondere auch bei kleineren Systemen mit hoher Kompaktheit.

Die vorliegende Erfindung wird nachfolgend anhand der beiliegenden Zeichnungen detailliert beschrieben, welche die vorliegende Erfindung nicht beschränken sollen.

Es zeigen:
- Figur 1a: eine Draufsicht auf die Vorderseite einer Ausführungsform aus dem Stand der Technik, basierend auf der Koaxialgeometrie;
- Figur 1b: das Strahlungsfeld der Anordnung von Figur 1 a;
- Figur 2a: eine dreidimensionale Ansicht einer weiteren beispielhaften Ausführungsform der vorliegenden Erfindung zur Veranschaulichung der räumlichen Geometrie;
- Figur 2b: eine Draufsicht auf die Vorderseite der in Figur 2a dargestellten beispielhaften Ausführungsform des Systems gemäß der vorliegenden Erfindung;
- Figur 2c: den Strahlengang der Anordnung von Figur 2a in schematischer Darstellung;
- Figur 3a und 3b: jeweils eine Draufsicht auf die Vorderseite weiterer beispielhafter Ausführungsformen des Systems gemäß der vorliegenden Erfindung; und
- Figur 4a und 4b: jeweils eine Draufsicht auf die Vorderseite weiterer beispielhafter Ausführungsformen des Systems gemäß der vorliegenden Erfindung.

Die verschiedenen in den Zeichnungen dargestellten Elemente sind nur repräsentativ und nicht notwendigerweise im Maßstab gezeichnet. Bestimmte Abschnitte hiervon können übertrieben sein, während andere minimiert sein können. Die Zeichnungen sollen beispielhafte Ausführungsformen der Offenbarung veranschaulichen, die vom Fachmann im Stand der Technik verstanden und geeigneterweise ausgeführt werden können. In den Figuren werden gleiche Komponenten und Elemente mit gleichen Bezugszeichen und Symbolen bezeichnet.

Die Figuren 1 a und 1 b wurden in der Beschreibungseinleitung bereits erläutert.

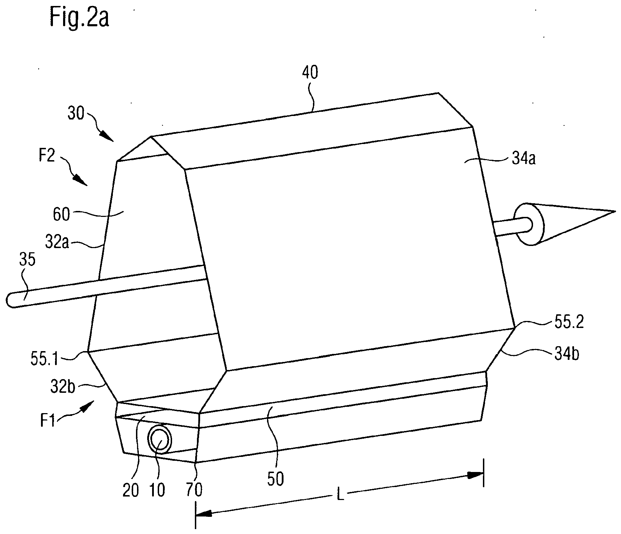

Figur 2a zeigt eine dreidimensionale Ansicht einer beispielhaften Ausführungsform der vorliegenden Erfindung zur Veranschaulichung der räumlichen Geometrie. Bei der gezeigten Ausführungsform der vorliegenden Erfindung handelt es sich um den sogenannten "modifizierten Eingangstrichter".

Der dargestellte Reaktor 30 ist im gezeigten Beispielfall ein Durchflussreaktor. Das verwendete Medium strömt durch den nach beiden Seiten offenen Innenraum 60 hindurch, von der Vorderseite zur Rückseite des Reaktors 30. Der Pfeil 35 symbolisiert die Fließrichtung innerhalb des Reaktors 30. Beispielsweise kann es sich um Wasser handeln. Es sind aber auch andere Medien möglich.

Die Seitenflächen 32a, 32b, 34a, 34b sowie das Abschlussteil 40 des Reaktors 30 sind jeweils als Reflektor ausgestaltet. Dies kann entweder durch entsprechende Auswahl des Wandmaterials oder durch Aufbringen einer Strahlungs-reflektierenden Schicht oder Beschichtung auf die Innen- oder Außenseite der Seitenwände 32a, 32b, 34a, 34b und des Abschlussteils 40 des Reaktors 30 erfolgen. Bei Vorliegen einer Außenschicht oder -beschichtung wird das Wandmaterial aus einem Strahlungs-transparenten Material, wie beispielsweise Strahlungs-transparentem Glas, ausgewählt. Somit wird der Reaktor 30 erfindungsgemäß bis auf einen Bereich im Eintrittsteil 50 des Reaktors 30 als Reflektor ausgeführt.

In der Fig. 2a befindet sich das Abschlussteil 40 oben im Reaktor 30 und das Eintrittsteil 50 unten im Reaktor 30; dies ist aber nicht zwingend erforderlich, andere Ausrichtungen sind ebenfalls möglich. Das Eintrittsteil 50 bezeichnet das die Seitenflächen 32b und 34b des Reaktors 30 verbindende Teil, das näher bei dem mindestens einen optischen System 10 angeordnet ist, also einen geringeren Abstand zum optischen System 10 aufweist (als das Abschlussteil 40). Das Abschlussteil 40 bezeichnet das die Seitenflächen 32a und 34a des Reaktors 30 verbindende andere Teil, welches von dem mindestens einen optischen System 10 entfernter angeordnet ist, also einen größeren Abstand zum optischen System 10 aufweist (als das Eintrittsteil 50).

Der modifizierte Eingangstrichter von Fig. 2a zeigt das Medium-führende Bauteil in Form des Reaktors 30, der in zwei Funktionsbereiche F1 und F2 unterteilt ist. Dabei wird der Reaktor 30 derart ausgestaltet, dass dieser aus einem ersten Funktionsbereich F1, welcher am nächsten zum mindestens einen optischen System 10 angeordnet ist, und einem zweiten Funktionsbereich F2, welcher vom mindestens einen optischen System in Form einer UV-Lichtquelle 10, weiter entfernt angeordnet ist, aufgebaut ist. Hierbei zeichnet sich der erste Funktionsbereich F1 dadurch aus, dass sich die Strahlung im Wesentlichen ungehindert ausbreitet und der zweite Funktionsbereich dadurch, dass im Wesentlichen Überlagerungen der Strahlung vorliegen.

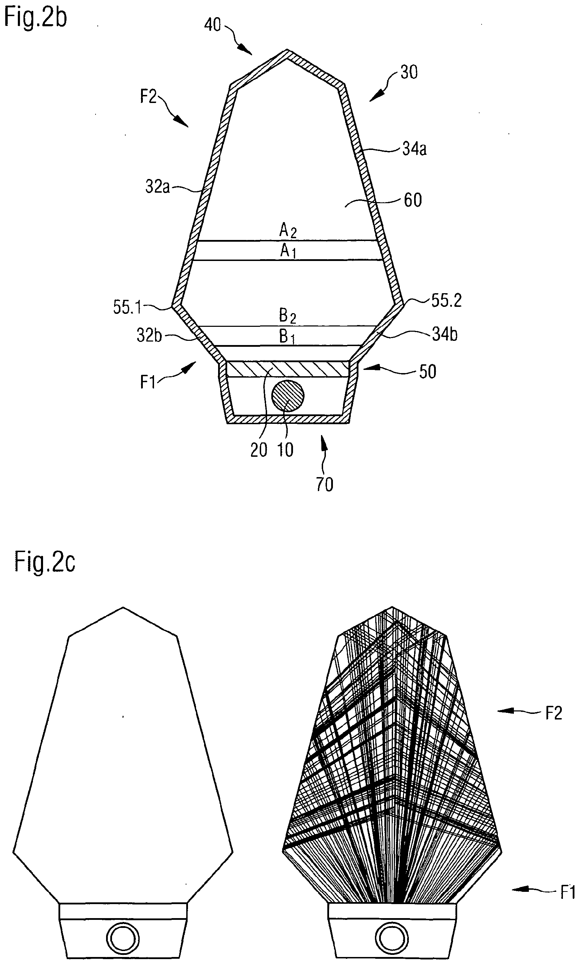

In der gezeigten Ausgestaltung gemäß der vorliegenden Erfindung ist der erste Funktionsbereich F1 so ausgestaltet, dass der Abstand zwischen den sich gegenüberliegenden Seitenflächen 32b und 34b des Reaktors 30 mit zunehmender Entfernung zum optischen System, beispielsweise in Form einer Lichtquelle 10, vorzugsweise kontinuierlich, zunimmt (Abstand B2 > Abstand B1, siehe Fig. 2b). Der zweite Funktionsbereich F2 ist so ausgestaltet, dass der Abstand zwischen den sich gegenüberliegenden Seitenflächen 32a und 34a des Reaktors 30 mit zunehmender Entfernung zum optischen System, beispielsweise in Form einer Lichtquelle 10, vorzugsweise kontinuierlich, abnimmt (Abstand A1 > Abstand A2, siehe Fig. 2b).

In der gezeigten Ausführungsform liegt ein baulicher Übergang 55 vor, bei dem der erste in den zweiten Funktionsbereich übergeht. Dies stellt hier jeweils eine Ecke bzw. Kante 55.1 und 55.2 dar, die die Seitenflächen jeweils unterteilt in 32a und 32b bzw. 34a und 34b.

Den Eintrittsteil 50 des Reaktors 30 bildet in der gezeigten Ausführungsform ein Strahlungs-transparenter Bereich in Form eines Strahlungs-transparenten Fensters 20, das die beiden Seitenflächen 32b und 34b miteinander verbindet und den Reaktor 30 abschließt. Durch dieses Strahlungs-transparente Fenster 20 kann das optische System 10 Licht in den Innenraum 60 des Reaktors 30 abstrahlen. Andere Geometrien, Formen und Größenverhältnisse des Fensters als die dargestellten sind möglich. Somit ist das optische System 10 außerhalb des Reaktors 30, im gezeigten Beispielfall unterhalb des Reaktors 30, angeordnet. Selbstverständlich können auch mehrere optische Systeme vorgesehen sein, die im gezeigten Beispielfall bevorzugt nebeneinander angeordnet wären. Im gezeigten Beispielfall ist das optische System 10 eine UV-Lichtquelle. Das erfindungsgemäße System ist hier daher ein UV-Desinfektionssystem. Dem optischen System in Form der UV-Lichtquelle 10 ist ein Reflektor (Lampenreflektor oder zweiter Reflektor) 70 zugeordnet, so dass in die falsche Richtung abgestrahltes Licht in den Reaktor 30 reflektiert wird.

Der Reflektor 70 könnte in der gezeigten Ausführungsform auch weggelassen werden. Vorteilhafterweise könnten dann vorzugsweise eine oder mehrere gerichtete UV-Lichtquellen 10 zum Einsatz kommen.

Das optische System 10 ist in der gezeigten beispielhaften Ausführungsform als UV-Stablampe dargestellt, die parallel zur Strömungsrichtung (Pfeil 35) außerhalb des Reaktors 30 angeordnet ist. Die UV-Stablampe erstreckt sich dabei über die gesamte Länge L des UV-Reaktors 30. Andere Bauweisen sind möglich. Die Zahl und Anordnung der UV-Lichtquellen ist beliebig variabel.

Eine Wandfläche des Reaktors 30 ist im gezeigten Beispielfall im Eintrittsteil 50 durch einen Strahlungs-transparenten Bereich 20, im vorliegenden Fall einen UV-transparenten Bereich 20, ersetzt. Der UV-transparente Bereich 20 ist im vorliegenden Ausführungsbeispiel daher zwischen der Lichtquelle 10 und dem Innenraum 60 des Reaktors 30 in Form eines UV-transparenten Fensters 20 vorgesehen. Das UV-transparente Material kann z.B. Glas sein. Durch das UV-transparente Fenster 20 wird die Lichtquelle 10 vor dem zu behandelnden Medium, das durch den Innenraum 60 des Reaktors 30 strömt, geschützt. Die Dimensionen des Fensters können auf die Dimensionen und Form des Reaktors 30 und das eingesetzte optische System 10 abgestimmt und an diese angepasst werden. Im gezeigten Beispielfall erstreckt sich der UV-transparente Bereich oder das UV-transparente Fenster 20 über die gesamte Länge L des Reaktors 30. Dies ist aber nicht in jedem Fall erforderlich. Andere Geometrien sind denkbar.

Die Dimensionen und die Form des UV-transparenten Bereichs oder Fensters 20 werden derart ausgewählt, dass die von der Lichtquelle 10 ausgesandte Strahlung in möglichst hohem Maße in den Innenraum 60 des Reaktors 30 eintreten kann.

Im gezeigten Beispielfall ist die Lichtquelle 10 eine UV-Stablampe, d.h. eine ungerichtete Lichtquelle. Für diesen Fall ist es besonders bevorzugt, einen Lampenreflektor 70 vorzusehen. Selbstverständlich kann erfindungsgemäß mehr als eine UV-Lichtquelle zum Einsatz kommen. Auch andere Lampentypen sind möglich. Beispielsweise könnten statt der UV-Stablampe auch UV-LEDs eingesetzt werden. Hierbei handelt es sich um gerichtete Lichtquellen, so dass ein Lampenreflektor in diesem Fall weggelassen werden könnte, ohne die erwünschte homogene Strahlungsverteilung zu gefährden.

Im dargestellten Ausführungsbeispiel der vorliegenden Erfindung ist der erste Reflektor, gebildet durch die Seitenflächen 32a, 32b, 34a, 34b und das Abschlussteil 40 unmittelbar in Kontakt mit dem zweiten Reflektor 70, d.h. er schließt sich unmittelbar an diesen an, so dass aus beiden ein Gesamtreflektor resultiert, durch den das zu desinfizierende Medium im Innenraum 60 hindurchströmt. Hieraus resultiert eine Kontaktkühlung des zweiten Reflektors 70.

Figur 2b zeigt eine Draufsicht auf die Vorderseite der in Figur 2a dargestellten beispielhaften Ausführungsform eines UV-Desinfektionssystems gemäß der vorliegenden Erfindung in Form eines modifizierten Eingangstrichters, wie diese bereits eingehend bei Fig. 2a erläutert wurde.

Fig. 2c stellt den Strahlengang der von einem optischen System in Form einer UV-Lichtquelle 10 ausgehenden Strahlung gemäß Fig. 2a oder 2b schematisch dar. Im Bereich direkt hinter dem Eintrittsfenster 20 breitet sich die eintretende Strahlung zunächst aus. In diesem Bereich (erster Funktionsbereich F1) nimmt die Intensität der Strahlung aufgrund der räumlichen Ausbreitung sowie einer möglichen Absorption durch das vorhandene Medium mit zunehmendem Abstand von der Strahlungsquelle 10 ab. Nach einer durch die Geometrie des Reaktors 30 festgelegten Wegstrecke trifft die Strahlung dann auf die reflektierenden Seitenwände 32a und 34a und wird unter einem Winkel zurückreflektiert. Dieser Winkel ist durch die Geometrie des Reaktors 30 derart bestimmt, dass im zweiten Funktionsbereich F2 im Wesentlichen eine Überlagerung der Strahlengänge stattfindet. Dadurch wird die Abschwächung der Strahlung durch Beiträge von den Wänden reflektierter Strahlen ausgeglichen, so dass die kumulierte Strahlungsintensität über den gesamten zweiten Funktionsbereich weitestgehend unverändert bleibt.

Die gezeigte Ecke bzw. Kante jeweils in der Seitenfläche stellt den Übergang vom ersten in den zweiten Funktionsbereich dar.

Anhand einer Simulation mit der sogenannten Ray Tracing-Methode werden für Figur 2a und 2b vom optischen System in Form einer Strahlungsquelle 10 ausgehende Strahlengänge berechnet, wobei die optischen Parameter der durchdrungenen Materialien, insbesondere Absorptions- und Reflexionskoeffizienten, berücksichtigt werden. Durch die Berechnung einer hohen Anzahl statistisch erzeugter Ausgangsstrahlen wird das resultierende Strahlungsfeld abgebildet.

Aus Fig. 2c geht daher hervor, dass die Strahlungsintensität über die gesamten Innenraum 60 des Reaktors 30 eine hohe Homogenität der Strahlungsintensität bereitstellt. Um dies zu quantifizieren wurde für die Anordnung von Fig. 2a und 2b eine Standardabweichung vom Mittelwert der Strahlungsdichte für den zweiten Funktionsbereich von 10% berechnet. Ein derartiger niedriger Wert belegt eine besonders hohe Strahlungshomogenität des Systems von Fig. 2a und 2b. Im ersten Funktionsbereich ist die Strahlung in der Nähe der Lichtquelle sehr stark, so dass für eine entsprechende Anwendung der erforderliche Strahlungswert in jedem Fall erreicht wird.

Wie ferner aus Fig. 2a hervorgeht, ist der Anteil des Volumens des zu desinfizieren Mediums, im vorliegenden Fall das Volumen des Innenraums 60, bezogen auf das Gesamtvolumen des Systems, sehr groß und beträgt über 80%.

Demzufolge weist diese Ausführungsform der vorliegenden Erfindung in Form eines UV-Desinfektionssystem, basierend auf der Form des modifizierten Eingangstrichters gemäß der Fig. 2a oder 2b, sowohl eine hohe Strahlungshomogenität als auch eine hohe Kompaktheit des Systems auf, wodurch eine verbesserte und damit insgesamt außerordentlich hohe Gesamteffektivität des Systems resultiert.

Figur 3a und 3b zeigen jeweils eine Draufsicht auf die Vorderseite weiterer beispielhafter Ausführungsformen des Systems gemäß der vorliegenden Erfindung. Es ist der modifizierte Eingangstrichter gezeigt, wobei die Seitenflächen 32a, 32b, 34a, 34b und das Abschlussteil 40 als Reflektor in verschiedenen Varianten ausgebildet sein können. In Fig.3a ist eine Strahlungs-reflektierende Innenschicht oder Beschichtung 65 und in Fig. 3b eine Strahlungs-reflektierende Außenschicht oder Beschichtung 67 vorgesehen. Das optische System 10 kann eine UV-Lichtquelle sein.

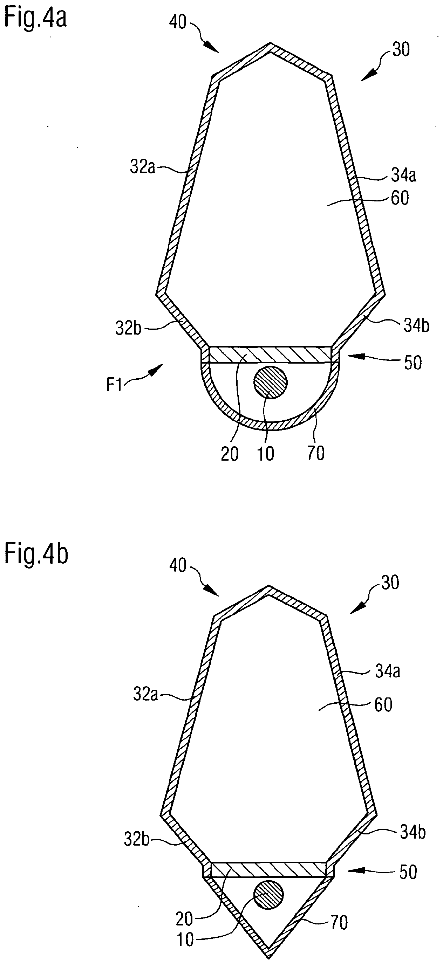

Figur 4a und 4b zeigen jeweils eine Draufsicht auf die Vorderseite weiterer beispielhaften Ausführungsformen des Systems gemäß der vorliegenden Erfindung, wobei die Form des Reflektors 70 variiert wurde. In Fig. 5a weist der Reflektor 70 eine runde Form auf. Es handelt sich im gezeigten Beispielfall um einen Hohlspiegel, der einen Kugelausschnitt darstellt. In Fig. 4b weist der Reflektor 70 eine eckige Form auf. Selbstverständlich sind auch andere Querschnitte und Geometrien mit einer anderen Zahl von optischen Systemen als die gezeigten möglich.

Die erfindungsgemäßen Systeme zeigen daher in unerwarteter Weise eine Strahlungsverteilung, die an keiner Stelle des Medium-führenden Innenraums im Reaktor Verarmungszonen zeigt, es können über den gesamten Reaktorquerschnitt relativ hohe Strahlungswerte erreicht werden. Zusätzlich wird eine besonders hohe Kompaktheit des Systems zur Verfügung gestellt.

Die Figuren 1 bis 4b verdeutlichen nur beispielhaft mögliche Ausgestaltungen. Diese sind nicht beschränkend zu verstehen, sondern stellen lediglich Beispiele

## Patentansprüche

1. System zur Behandlung von Gasen und/oder Flüssigkeiten mit UV-Strahlung, umfassend zumindest eine UV-Lichtquelle (10) sowie einen Reaktor (30), der Seitenflächen (32, 32a, 32b, 32.1, 32.2, 32.3, 32.4, 34, 34a, 34b, 34.1, 34.2, 34.3, 34.4), ein erstes die Seitenflächen verbindendes Teil oder Abschlussteil (40, 40.1, 40.2, 40.3, 40.4) sowie einen zur Vorder- und Rückseite offenen Innenraum (60, 60.1, 60.2, 60.3, 60.4) umfasst, durch den das Medium strömt, wobei der Reaktor (30) zumindest teilweise in Form eines Reflektors ausgebildet ist, der vom optischen System (10) ausgesandte Strahlung in den Innenraum (60) des Reaktors (30) reflektiert, wobei der Reaktor (30) in zwei Funktionsbereiche unterteilt ist, einen ersten Funktionsbereich F1, welcher dem mindestens einen optischen System (10) am nächsten liegt, und einen zweiten Funktionsbereich F2, welcher von dem mindestens einen optischen System (10) weiter entfernt angeordnet ist als der erste Funktionsbereich F1, wobei sich im Betriebszustand des Systems Strahlung im ersten Funktionsbereich ungehindert ausbreiten kann und im zweiten Funktionsbereich Überlagerungen der Strahlung vorliegen und
a) im ersten Funktionsbereich F1 des Reaktors (30) der Abstand zwischen den sich gegenüberliegenden Seitenflächen (32b und 34b) des Reaktors sich mit zunehmender Entfernung zu dem mindestens einen optischen System vergrößert,
**dadurch gekennzeichnet, dass**
der Querschnitt des Reaktors (30) die Form eines unregelmäßigen Vielecks aufweist und
ein UV-transparenter Bereich (20) vorgesehen ist, der gemäß Variante a) in Form eines UV-transparenten Fensters am Eintrittsteil (50) des Reaktors vorgesehen ist.

2. System nach Anspruch 1, **dadurch gekennzeichnet, dass** die Seitenflächen (32, 32a, 32b, 32.1, 32.2, 32.3, 32.4, 34, 34a, 34b, 34.1, 34.2, 34.3, 34.4) und das Abschlussteil (40, 40.1, 40.2, 40.3, 40.4) als Reflektor ausgebildet sind.

3. Verwendung eines Systems nach einem der Ansprüche 1 oder 2 als UV-Desinfektionssystem.

## Claims

1. A system for the treatment of gases and/or liquids with UV radiation, comprising at least one UV light source (10) and a reactor (30) having side surfaces (32, 32a, 32b, 32.1, 32.2, 32.3, 32.4, 34, 34a, 34b, 34.1, 34.2, 34.3, 34.4), a first part or end part (40, 40.1, 40.2, 40.3, 40.4) connecting the side surfaces, as well as an inner space (60, 60.1, 60.2, 60.3, 60.4) which is open towards the front and rear side, through which the medium flows, wherein the reactor (30) is at least partially formed in the form of a reflector which reflects radiation emitted by the optical system (10) into the inner space (60) of the reactor (30), wherein the reactor (30) is divided into two functional areas, a first functional area F1, which is closest to the at least one optical system (10), and a second functional area F2, which is arranged further away from the at least one optical system (10) than the first functional area F1, wherein radiation can propagate unhindered in the first functional area in the operating state of the system and superpositions of the radiation are present in the second functional area, and
a) in the first functional area F1 of the reactor (30), the distance between the opposing side surfaces (32b and 34b) of the reactor increases with increasing distance from the at least one optical system,
**characterized in that**
the cross-section of the reactor (30) is in the form of an irregular polygonal, and
a UV-transparent region (20) is provided, which according to variant a) is provided in the form of a UV-transparent window at the inlet part (50) of the reactor.

2. A system according to claim 1, **characterized in that** the side surfaces (32, 32a, 32b, 32.1, 32.2, 32.3, 32.4, 34, 34a, 34b, 34.1, 34.2, 34.3, 34.4) and the end part (40, 40.1, 40.2, 40.3, 40.4) are designed as reflectors.

3. Use of a system according to one of claims 1 or 2 as a UV disinfecting system.

## Revendications

1. Système pour le traitement de gaz et/ou de liquides avec un rayonnement UV, comprenant au moins une source de lumière UV (10) et un réacteur (30) qui comprend des surfaces latérales (32, 32a, 32b, 32.1, 32.2, 32.3, 32.4, 34, 34a, 34b, 34.1, 34.2, 34.3, 34.4), une première partie reliant les surfaces latérales ou partie de fermeture (40, 40.1, 40.2, 40.3, 40.4) et un espace intérieur (60, 60.1, 60.2, 60.3, 60.4) ouvert du côté avant ou arrière, à travers lequel circule le fluide, dans lequel le réacteur (30) est au moins partiellement construit sous la forme d'un réflecteur qui réfléchit le rayonnement émis par le système optique (10) dans l'espace intérieur (60) du réacteur (30), dans lequel le réacteur (30) est partagé en deux zones fonctionnelles, une première zone fonctionnelle F1 qui est la plus proche de l'au moins un système optique (10) et une deuxième zone fonctionnelle F2 qui est plus éloignée de l'au moins un système optique (10) que la première zone fonctionnelle F1, le rayonnement pouvant se propager sans obstacle dans la première zone fonctionnelle dans l'état de fonctionnement du système et des superpositions au rayonnement se produisant dans la deuxième zone fonctionnelle, et
a) la distance entre les surfaces latérales (32b et 34b) du réacteur qui se font face dans la première zone fonctionnelle F1 du réacteur (30) augmentant avec la distance par rapport à l'au moins un système optique,
**caractérisé en ce que**
la section du réacteur (30) a une forme quadrangulaire irrégulière et
il est prévu une zone transparente aux UV (20) qui est prévue, selon la variante a), sous la forme d'une fenêtre transparente aux UV dans la partie d'entrée (50) du réacteur.

2. Système selon la revendication 1, **caractérisé en ce que** les surfaces latérales (32, 32a, 32b, 32.1, 32.2, 32.3, 32.4, 34, 34a, 34b, 34.1, 34.2, 34.3, 34.4) et la partie de fermeture (40, 40.1, 40.2, 40.3, 40.4) sont réalisées comme un réflecteur.

3. Utilisation d'un système selon l'une des revendications 1 ou 2 comme système de désinfection aux UV.
